# EUROPEAN PATENT APPLICATION

(11) **EP 4 317 132 A1**
(43) Date of publication of application: **07.02.2024**
(21) Application number: 22774926.4
(22) Date of filing: 28.02.2022
(51) Int. Cl.: C07C 407/00, C07C 409/10, C07B 61/00

(54) **PRODUCTION EQUIPMENT AND PRODUCTION METHOD FOR CUMENE HYDROPEROXIDE**

(30) Priority: 24.03.2021 JP 2021050160
(71) Applicant: Sumitomo Chemical Company Limited, Tokyo 103-6020 (JP)
(72) Inventor: HASHIZUME Satoru, Ichihara-shi, Chiba 299-0195 (JP); INUI Yuki, Ichihara-shi, Chiba 299-0195 (JP)
(74) Representative: Vossius & Partner Patentanwälte Rechtsanwälte mbB
(86) International application number: PCT/JP2022/008318
(87) International publication number: WO 2022/202128

(57) **Abstract**

Production equipment for oxidizing cumene to produce cumene hydroperoxide, said equipment comprising: a device A that oxidizes cumene to obtain cumene hydroperoxide; and a heat exchange system X, wherein the heat exchange system X comprises a medium capable of both heating and cooling the oxidation reaction system, a medium cooling unit capable of cooling the medium, and a medium heating unit capable of heating the medium.

## Description

### TECHNICAL FIELD

The present invention relates to production equipment and a production method for cumene hydroperoxide. More particularly, the present invention relates to production equipment and a production method for cumene hydroperoxide meant for efficiently starting up a step of oxidizing cumene to obtain cumene hydroperoxide in a system for producing propylene oxide by a continuous method.

### BACKGROUND ART

A method for obtaining propylene oxide by reacting cumene hydroperoxide with propylene is well known. The resulting propylene oxide is purified by being subjected to a purification step.

### PRIOR ART DOCUMENT

### PATENT DOCUMENT

**Patent Document 1:** JP 2007-284419 A

### SUMMARY OF INVENTION

### TECHNICAL PROBLEM

A reaction of oxidizing cumene to produce cumene hydroperoxide is an exothermic reaction. On that account, during steady operation (steady state) in a continuous method, it is preferable to cool a reaction system using a medium in such a manner that the temperature in the reaction system does not rise too high. On the other hand, before the reaction reaches steady operation, that is, at the time of startup, it is preferable to heat the reaction system in order to make the reaction system be in a steady state more quickly.

It is an object of the present application to provide production equipment and a production method for cumene hydroperoxide, which makes it possible for both to efficiently start up a step of oxidizing cumene to obtain cumene hydroperoxide and to safely carry out steady operation.

### SOLUTION TO PROBLEM

The present invention relates to the following but is not limited thereto.

### [Invention 1]

Production equipment for producing cumene hydroperoxide by oxidizing cumene, comprising:
A: a device that oxidizes cumene to obtain cumene hydroperoxide; and
X: a heat exchange system,
wherein the heat exchange system X comprises a medium capable of both heating and cooling a reaction system for the oxidation, a medium cooling unit capable of cooling the medium, and a medium heating unit capable of heating the medium.

### [Invention 2]

The production equipment according to Invention 1, wherein the medium is a fluid containing water.

### [Invention 3]

The production equipment according to Invention 1 or 2, comprising
B: a device that allows cumene hydroperoxide to react with propylene to obtain propylene oxide and cumyl alcohol; and
E: a device that converts cumyl alcohol into cumene.

### [Invention 4]

The production equipment according to any of Inventions 1 to 3, wherein the cooling of the medium is carried out by a fluid containing at least one selected from water, a process fluid of the device B, and a process fluid of the device E.

### [Invention 5]

The production equipment according to any of Inventions 1 to 4, comprising at least one selected from
C: a device that separates propylene;
D: a device that separates propylene oxide; and
F: a device that purifies cumene.

### [Invention 6]

The production equipment according to Invention 5, wherein the cooling of the medium is carried out by a fluid containing at least one selected from water, a process fluid of the device C, a process fluid of the device D, and a process fluid of the device F.

### [Invention 7]

The production equipment according to Invention 6, wherein the cooling of the medium is carried out by a fluid containing at least one selected from water, a process fluid of the device C, and a process fluid of the device D.

### [Invention 8]

The production equipment according to Invention 7, wherein the device C comprises a device C' that separates propane, and the cooling of the medium is carried out by a fluid containing a process fluid of the device C'.

### [Invention 9]

The production equipment according to Invention 7 or 8, wherein the device D comprises a device D' that separates a heavy component, and the cooling of the medium is carried out by a fluid containing a process fluid of the device D'.

### [Invention 10]

The production equipment according to any of Inventions 7 to 9, wherein the device D comprises a device D" that separates a light component, and the cooling of the medium is carried out by a fluid containing a process fluid of the device D".

### [Invention 11]

The production equipment according to any of Inventions 1 to 10, wherein the heating of the medium is carried out by a fluid containing at least one selected from water (liquid) and water vapor.

### [Invention 12]

The production equipment according to any of Inventions 1 to 11, comprising another cooling means than the heat exchange system X as a means for cooling the reaction system for the oxidation.

### [Invention 13]

The production equipment according to Invention 12, wherein the cooling means is an air fin cooler.

### [Invention 14]

A method for producing cumene hydroperoxide, comprising the following steps:
a step of oxidizing cumene to obtain cumene hydroperoxide;
a step of heating a reaction system for the oxidation with a medium x;
a step of heating, in a heat exchange system X, the medium x having been cooled by the aforementioned heating;
a step of cooling the reaction system for the oxidation with the medium x; and
a step of cooling, in the heat exchange system X, the medium x having been heated by the aforementioned cooling.

### ADVANTAGEOUS EFFECTS OF INVENTION

According to the present invention, a reaction system can be heated when a step of oxidizing cumene to obtain cumene hydroperoxide is started up, and after the reaction system is sufficiently heated, the reaction system can also be cooled in order to suppress overheating, and due to them, not only can the step be efficiently started up, but also the temperature of the reaction system can be maintained at a prescribed temperature, thereby enabling continuous operation.

### BRIEF DESCRIPTION OF DRAWINGS

Fig. 1 shows an example of the cumene hydroperoxide production equipment of the present invention.
Fig. 2 shows an example of a heat exchange system comprised in the cumene hydroperoxide production equipment of the present invention.

### DESCRIPTION OF EMBODIMENTS

### Definition

All numbers disclosed in the present specification are approximate values whether or not the word "about" or "approximate" is used in connection therewith or not. These numbers may vary by 1 percent, 2 percent, 5 percent, or sometimes 10 to 20 percent. Whenever a range of numerical values accompanied by a lower limit R^{L} and an upper limit R^{U} is disclosed, arbitrary numbers included within the range are specifically disclosed. In particular, the following numbers within the range are specifically disclosed. R=R^{L}+k*(R^{U}-R^{L}), where k is a variable ranging from 1 percent to 100 percent in 1 percent increments, *i.e.,* k is 1 percent, 2 percent, 3 percent, 4 percent, 5 percent, ... 50 percent, 51 percent, 52 percent, ... 95 percent, 96 percent, 97 percent, 98 percent, 99 percent, or 100 percent). Moreover, ranges of arbitrary numerical values defined by numbers of two R as set forth above are also specifically disclosed.

The description "lower limit to upper limit", which represents a numerical value range represents "lower limit or more and upper limit or less", and the description "upper limit to lower limit" represents "upper limit or less and lower limit or more". That is to say, these descriptions represent numerical value ranges including a lower limit and an upper limit, but in one embodiment, one or both of the upper limit and the lower limit may be excluded, that is, "lower limit to upper limit" may represent "more than lower limit and upper limit or less", "lower limit or more and less than upper limit", or "more than lower limit and less than upper limit". Furthermore, "- or more" and "- or less" may represent "more than -" and "less than -", respectively.

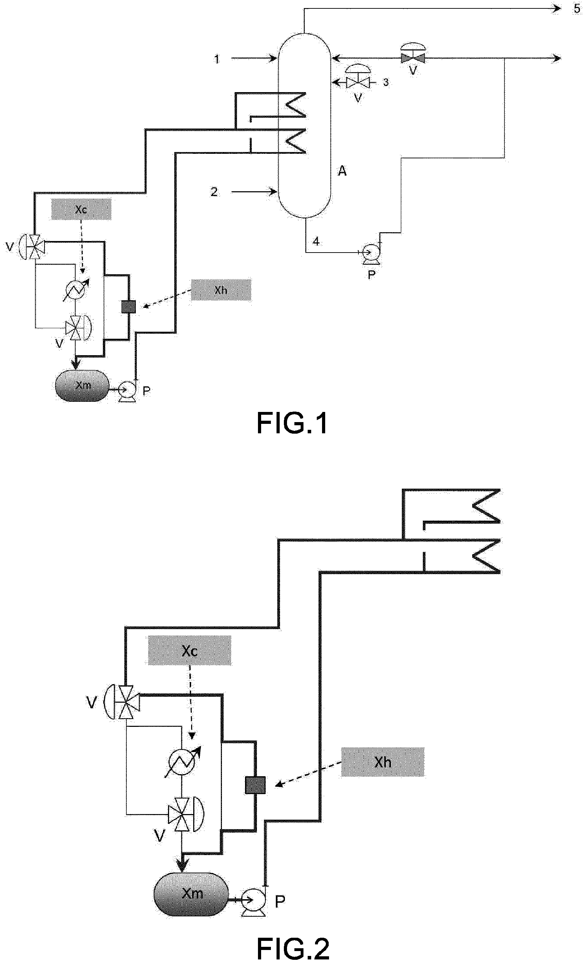

Some embodiments of the present invention will be described in detail with reference to Fig. 1. However, the present invention is in no way limited to the following embodiments.

### Cumene hydroperoxide production equipment

Oxidation of cumene (1) is usually carried out by autoxidation due to an oxygen-containing gas (2) such as air or oxygen concentrated air. In particular, an emulsification oxidation method in water/alkaline emulsion is preferable from the viewpoint of enhancing yield of cume hydroperoxide. A usual reaction temperature is 50 to 200°C, and a reaction pressure is between atmospheric pressure and 5 MPa. In the case of the emulsification oxidation method, an alkali metal compound such as NaOH or KOH, an alkaline earth metal compound, an alkali metal carbonate such as Na₂CO₃ or NaHCO₃, ammonia, NH₄CO₃, an alkali metal ammonium carbonate, or the like are used as an alkaline reagent (3).

The cumene hydroperoxide production equipment of the present invention comprises:
A: a device that oxidizes cumene to produce cumene hydroperoxide; and
X: a heat exchange system.

The heat exchange system X comprises a medium (Xm), a medium heating unit (Xh), and a medium cooling unit (Xc). The medium is capable of both heating and cooling a reaction system for the oxidation. The medium heating unit is capable of heating the medium to a temperature higher than that of the reaction system, and the heated medium is capable of heating the reaction system. The medium cooling unit is capable of cooling the medium to a temperature lower than that of the reaction system, and the cooled medium is capable of cooling the reaction system. The "reaction system" may be referred to as "reaction mixture", "reaction device contents", "production device contents", "reaction device interior", or "production device interior".

In one embodiment, the medium is a fluid containing water. The "water" is selected from liquid water, water vapor, and a mixture thereof.

In one embodiment, the cumene hydroperoxide production equipment of the present invention comprises the following devices:
B: a device that allows cumene hydroperoxide to react with propylene to obtain propylene oxide and cumyl alcohol; and
E: a device that converts cumyl alcohol into cumene.
Here, cumyl alcohol refers to 2-phenyl-2-propanol.

In one embodiment, the cumene hydroperoxide (accurately, a fluid (4) containing cumene hydroperoxide) obtained in the device A may be used in the device B, and the cumyl alcohol obtained in the device B may be used in the device E. In the present specification, for example, "the cumene hydroperoxide obtained in the device A and used in the device B" not only means that the cumene hydroperoxide obtained in the device A is directly introduced into the device B but also means that the cumene hydroperoxide obtained in the device A is subjected to treatments such as separation/purification in one or a plurality of devices, and thereafter, the cumene hydroperoxide having been subjected to the treatments is introduced into the device B.

The same shall apply to other steps.

Preferably, the production equipment comprises, in addition to the above, at least one selected from the following devices:
C: a device that separates propylene;
D: a device that separates propylene oxide; and
F: a device that purifies cumene.

When the production equipment of the present invention comprises the device C, propylene that is unreacted in the device B is separated in the device C, and cumyl alcohol having been improved in purity is obtained. The cumyl alcohol obtained in the device C may be used in the device E. The propylene to be separated in the device C may be used in the device B.

Accompanying the device C, the production equipment of the present invention may comprise a device that further purifies the separated propylene, and the purified propylene may be used in the device B.

When the production equipment of the present invention comprises the device D, the propylene oxide obtained in the device B may be separated in the device D, and preferably, the device D is comprised in the production device of the present invention together with the device C, and a mixture containing cumyl alcohol obtained in the device C, the mixture containing propylene oxide, is sent to the device D, and the propylene oxide is separated, thereby obtaining cumyl alcohol having been more improved in purity. The cumyl alcohol obtained in the device D may be used in the device E.

Accompanying the device D, the production equipment of the present invention may comprise a device that further purifies the separated propylene oxide.

When the production equipment of the present invention comprises the device F, cumene is purified by separating a by-product obtained together with cumene in the device E. The cumene obtained in the device F, that is, cumene having been improved in purity, may be used in the device A.

In one embodiment, cooling of the medium is carried out by a fluid containing at least one selected from water, a process fluid of the device B, and a process fluid of the device E. In one embodiment, the cooling is carried out by a fluid containing at least one selected from water, a process fluid of the device C, a process fluid of the device D, and a process fluid of the device F. Preferably, the cooling is carried out by a fluid containing at least one selected from water, a process fluid of the device C, and a process fluid of the device D. Here, "a process fluid of a device Y" means a part or all fluid taken out of the device Y. When the process fluid is utilized as a medium of the heat exchange system, the fluid may be moved forward to a next device (column) after utilized or may be returned to the same device (column). In one embodiment, cooling of the medium is carried out by a further heat exchange system.

In the cooling of the medium, none of the cooling start temperature (medium temperature before cooling) and the cooling end temperature (medium temperature after cooling) are limited, but it is preferable to set the cooling start temperature to 30 to 180°C, and it is preferable to set the cooling end temperature to 25 to 175°C.

In one embodiment, the device C that separates propylene comprises a number of devices (columns), and preferably, the columns are different in separation or purification conditions. In one embodiment, the device C comprises a device (column) C' that separates propane. This may be referred to as "a propane separation device (column)".

In one embodiment, the cooling of the medium is carried out by a fluid containing a process fluid from any of the columns comprised in the device C, preferably from the propane separation device (column) C'.

In one embodiment, the device D that separates propylene oxide comprises a number of devices (columns), and preferably, the columns are different in separation or purification conditions. In one embodiment, the device D comprises a number of devices (columns) that separates a number of components that are different in boiling point, and preferably comprises a device (column) D' that separates a heavy component and a device (column) D" that separates a light component. These may be referred to as "a heavy component separation device (column)" and "a light component separation device (column)". Here, a component having a relatively high boiling point is referred to as "a heavy component", and a component having a relatively low boiling point is referred to as "a light component". The "light component separation device (column)" may be referred to as "a light fraction cutting device (column)".

In one embodiment, cooling of the medium is carried out by a fluid containing a process fluid from any of columns comprised in the device D, for example, from the heavy component separation device (column) D' and/or the light component separation device (column) D".

In one embodiment, the heating of the medium is carried out by a further heat exchange system. Preferably, the heating is carried out by a fluid containing at least one selected from water (liquid) and water vapor.

In the heating of the medium, none of a heating start temperature (medium temperature before heating) and a heating end temperature (medium temperature after heating) are limited, but it is preferable to set the heating start temperature to 20 to 150°C, and it is preferable to set the heating end temperature to 25 to 155°C.

In one embodiment, the cumene hydroperoxide production equipment of the present invention further comprises another means capable of cooling the reaction system for the cumene oxidation reaction. Said another cooling means is, for example, an air fin cooler. The air fin cooler is, for example, a natural draft air fin cooler.

The present application also provides an invention related to a method for producing cumene hydroperoxide, which goes along with the description of the cumene hydroperoxide production equipment of the present invention.

The aforementioned cumene hydroperoxide production equipment is preferably adopted in a method for producing propylene oxide described below.

In one embodiment, the method for producing propylene oxide includes the following steps:
step a: a step of oxidizing cumene to produce cumene hydroperoxide, and
step b: a step of reacting the cumene hydroperoxide obtained in the step a with propylene to obtain a fluid b' containing propylene oxide and cumyl alcohol.

The step b is carried out using the aforementioned device B. Hereinafter, the reaction of cumene hydroperoxide with propylene in the step b is sometimes described as "epoxidation reaction".

In the step b, it is preferable to react cumene hydroperoxide with propylene in the presence of a catalyst containing titanium-containing silicon oxide, from the viewpoint of highly selectively obtaining propylene oxide in a high yield. The catalyst is preferably a so-called Ti-silica catalyst containing Ti that is chemically bonded to silicon oxide. Examples thereof include a catalyst wherein a Ti compound is supported on a silica carrier, a catalyst combined with silicon oxide through a coprecipitation method or a sol-gel method, and a zeolite compound containing Ti.

The epoxidation reaction can be carried out using a solvent in a liquid phase. The solvent should be a liquid at a temperature and a pressure during reaction and should be substantially inert to a reactant and a product. An example of the solvent is cumene.

The epoxidation reaction temperature is generally 0 to 200°C, but it is preferably 25 to 200°C. The reaction pressure may be a pressure enough to keep the reaction mixture in a state of a liquid. In general, a pressure of 100 to 10000 kPa-G (gauge pressure) is advantageous.

The epoxidation reaction can be advantageously carried out using a catalyst in the form of a slurry or fixed bed. In the case of large-scale industrial operations, it is preferable to use a fixed bed. The epoxidation reaction can be carried out by a batch method, a semi-continuous method, or a continuous method.

The fluid b' obtained in the step b usually contains unreacted propylene.

In one embodiment, the method for producing propylene oxide includes, in addition to the steps a and b, the following step:
step c: a step of separating propylene from the fluid b' obtained in the step b to obtain a fluid c' containing propylene oxide and cumyl alcohol.
The step c is carried out using the aforementioned device C. The fluid c' may contain a small amount of propylene. A propylene concentration in the fluid c' is lower than a propylene concentration in the fluid b'.

In one embodiment, the method for producing propylene oxide includes, in addition to the steps a, b, and c, the following steps:
step d: a step of separating propylene oxide from the fluid c' obtained in the step c to obtain a fluid d' containing cumyl alcohol, and/or
step e: a step of converting cumyl alcohol in the fluid c' obtained in the step c or the fluid d' obtained in the step d into cumene to obtain a fluid e' containing cumene.
The step d is carried out using the aforementioned device D. A cumyl alcohol concentration in the fluid d' is higher than a cumyl alcohol concentration in the fluid c'.

The step e is carried out using the aforementioned device E. Examples of methods for converting the cumyl alcohol into cumene include (e1) a method of hydrocracking cumyl alcohol to obtain cumene, and (e2) a method of dehydrating cumyl alcohol and then hydrogenating it to obtain cumene.

In the case of (e1), in a reactor, a raw material fluid (fluid c' or fluid d') and hydrogen are brought into contact with a catalyst to react cumyl alcohol in the raw material fluid with hydrogen, thereby obtaining a fluid e' containing cumene.

Examples of the catalysts used in the hydrocracking reaction (sometimes described as "hydrocracking catalysts" hereinafter) include catalysts containing metals of Group 9, Group 10, Group 11, or Group 12 of the periodic table, and specific examples thereof include a catalyst containing cobalt, a catalyst containing nickel, a catalyst containing palladium, a catalyst containing copper, and a catalyst containing zinc. From the viewpoint of suppressing production of a by-product, a catalyst containing nickel, a catalyst containing palladium, or a catalyst containing copper is preferable. Examples of the catalysts containing nickel include nickel, nickel-alumina, nickel-silica, and nickel-carbon; examples of the catalysts containing palladium include palladium-alumina, palladium-silica, and palladium-carbon; and examples of the catalysts containing copper include copper, Raney copper, copper-chromium, copper-zinc, copper-chromium-zinc, copper-silica, and copper-alumina.

The reactor used for the hydrocracking reaction contains any one or a combination of a number of the catalysts. The reactor can be in the form of a slurry bed or a fixed bed. In the case of large-scale industrial operations, it is preferable to use a fixed bed. The reaction is preferably carried out by a continuous method.

The amount of hydrogen consumed in the hydrocracking reaction is equimolar with cumyl alcohol. However, in the raw material liquid, components other than cumyl alcohol that consumes hydrogen are usually contained, and therefore, from the viewpoint of ensuring the conversion rate of the cumyl alcohol, it is preferable to feed hydrogen excessively compared with the stoichiometric amount. As the hydrogen partial pressure is increased, the reaction proceeds more rapidly. Therefore, a hydrogen/cumyl alcohol mole ratio is usually adjusted to 1/1 to 20/1, is preferably 1/1 to 10/1, and is more preferably 1/1 to 5/1. A hydrogen/(cumene+cumyl alcohol) mole ratio is usually 1/25 or more.

Excess hydrogen remaining after the hydrocracking reaction can also be recycled and used after it is separated from the reaction liquid. The hydrocracking reaction temperature is usually 0 to 500°C, but it is preferably 50 to 450°C, and more preferably 150 to 300°C. The hydrocracking reaction pressure is usually 100 to 10000 kPa-G, preferably 500 to 4000 kPa-G, and more preferably 1000 to 2000 kPa-G.

When the hydrocracking reaction is applied, the conversion rate of the cumyl alcohol is usually 90% or more.

In the case of (e2), in a reactor, a raw material fluid (fluid c' or fluid d') is brought into contact with a catalyst, and by the dehydration reaction of cumyl alcohol in the raw material fluid, a fluid containing α-methylstyrene is obtained, and subsequently, the fluid containing α-methylstyrene and hydrogen are brought into contact with a catalyst in the reactor to subject α-methylstyrene and hydrogen to hydrogenation reaction, thereby obtaining a fluid e' containing cumene.

In the present embodiment, the step (reaction) of dehydrating cumyl alcohol to obtain a fluid containing α-methylstyrene is sometimes described as "dehydration step (dehydration reaction)", and the step (reaction) of subjecting a fluid containing α-methylstyrene and hydrogen to hydrogenation reaction to obtain a fluid e' containing cumene is sometimes described as "hydrogenation step (hydrogenation reaction)".

Examples of the catalysts used in the dehydration step (sometimes described as "dehydration catalyst" hereinafter) include homogeneous acid catalysts, such as sulfuric acid, phosphoric acid, and p-toluenesulfonic acid; and solid acid catalysts, such as activated alumna, titania, zirconia, silica alumina, and zeolite.

From the viewpoint of enhancing reaction efficiency, it is preferable to carry out the dehydration step in the presence of a solid acid catalyst and is more preferable to use activated alumina.

The dehydration reaction in the dehydration step is usually carried out by bringing the fluid containing cumyl alcohol into contact with the dehydration catalyst in the reactor. In order to carry out the hydrogenation reaction in the hydrogenation step subsequently to the dehydration reaction, the liquid containing cumyl alcohol may be brought into contact with the dehydration catalyst in the presence of hydrogen. The dehydration reaction temperature is usually 50 to 450°C, but it is preferably 150 to 300°C. The dehydration reaction pressure is usually 10 to 10000 kPa-G.

The catalyst used in the hydrogenation step (sometimes described as "hydrogenation catalyst" hereinafter) is, for example, a catalyst containing a metal of Group 10 or Group 11 of the periodic table, and specific examples thereof include a catalyst containing nickel, a catalyst containing palladium, a catalyst containing platinum, and a catalyst containing copper. From the viewpoints of suppression of nucleus hydrogenation reaction of an aromatic ring, and a high yield, a catalyst containing nickel, a catalyst containing palladium, or a catalyst containing copper is preferable. The catalyst containing nickel is preferably nickel, nickel-alumina, nickel-silica, or nickel-carbon; the catalyst containing palladium is preferably palladium-alumina, palladium-silica, or palladium-carbon; and the catalyst containing copper is preferably copper, Raney copper, copper-chromium, copper-zinc, copper-chromium-zinc, copper-silica, or copper-alumina. These catalysts can be used individually or in combination of a number thereof.

The hydrogenation step is carried out by bringing the fluid containing α-methylstyrene and hydrogen into contact with the hydrogenation catalyst in the reactor. Subsequently to the aforementioned dehydration reaction, the hydrogenation reaction is carried out, and in this embodiment, a part of water generated in the dehydration reaction may be separated by oil-water separation or the like, or may be brought into contact with the hydrogenation catalyst together with the α-methylstyrene, without being separated.

The amount of hydrogen consumed in the hydrogenation reaction is equimolar with α-methylstyrene. However, in the raw material liquid, components other than α-methylstyrene that consumes hydrogen are usually contained, and therefore, from the viewpoint of ensuring the conversion rate of the α-methylstyrene, it is preferable to feed hydrogen excessively compared with the stoichiometric amount. As the hydrogen partial pressure is increased, the reaction proceeds more rapidly. Therefore, a hydrogen/α-methylstyrene mole ratio is usually adjusted to 1/1 to 20/1, is preferably 1/1 to 10/1, and is more preferably 1/1 to 5/1. Excess hydrogen remaining after the hydrogenation reaction can also be recycled and used after it is separated from the reaction liquid. A hydrogen/(cumene+cumyl alcohol) mole ratio is usually 1/25 or more. In the case of (b), the amount of substance of "hydrogen" in the mole ratio is an amount of substance of hydrogen that is subjected to the hydrogenation reaction, and the amount of substance of "cumene+cumyl alcohol" is an amount of substance of the total of cumene and cumyl alcohol in the liquid that is subjected to the dehydration reaction.

The hydrogenation reaction temperature is usually 0 to 500°C, but it is preferably 30 to 400°C, and more preferably 50 to 300°C. The hydrogenation reaction pressure is usually 100 to 10000 kPa-G.

The dehydration reaction and the subsequent hydrogenation reaction may be carried out in a reactor in which a dehydration catalyst and a hydrogenation catalyst are contained in one container in this order from the upstream side, may be carried out in a reactor in which a catalyst obtained by physically mixing a dehydration catalyst and a hydrogenation catalyst is contained in one container, may be carried out in a reactor in which a hydrogenation catalyst supported on a dehydration catalyst is contained in one container, or may be carried out in a reactor in which a container containing a dehydration catalyst and a container containing a hydrogenation catalyst are connected in series in this order from the upstream side through a line.

The state of contact of the catalyst with the fluid in the container can be in the form of a slurry bed or a fixed bed. In the case of large-scale industrial operations, it is preferable to use a fixed bed.

In one embodiment, the method for producing propylene oxide includes, in addition to the steps a, b, c and/or d, and e, the following step:
step f: a step of purifying cumene in the fluid e' obtained in the step e to obtain a fluid f containing purified cumene.

The step f is carried out using the aforementioned device F. A cumene concentration in the fluid f is higher than a cumene concentration in the fluid e'. In the step f, impurities produced as by-products in at least one step selected from the group consisting of the steps a, b, c, d, and e can be removed from the fluid e'. Examples of the impurities include acetophenone, a cumene dimer, ethylbenzene, and phenols. Here, the cumene dimer refers to a compound typified by 2,3-dimethyl-2,3-diphenylbutane, dicumyl ether, dicumyl peroxide, or the like.

The purification of cumene is carried out by distillation and water washing or the like. The purified cumene may be recycled for the step a.

### Step a at the time of startup

At the time of startup, the step a may comprise the following steps:
step a1: a step of packing a fluid containing cumene in the device A;
step a2: a step of heating the fluid containing cumene, which has been packed in the step a1, by a heat exchange system X to increase a temperature of a reaction system in the device A up to a prescribed temperature;
step a3: a step of feeding a fluid containing oxygen to the device A, starting oxidation reaction of cumene, and increasing a cumene hydroperoxide concentration in the reaction system to a prescribed concentration; and
step a4: a step of discharging a fluid a' containing cumene hydroperoxide from the device A, and feeding the fluid a' to the device B.

In the steps a1, a2, and a3, it is preferable that the reaction liquid should not be discharged from the device A before the cumene hydroperoxide concentration in the reaction system rises up to a prescribed concentration.

In the step a1, the fluid containing cumene may contain 0.001 to 20 wt% of cumene hydroperoxide.

In the step a2, the temperature of the reaction system in the device A is preferably increased up to 80 to 95°C.

In the step a3, the cumene hydroperoxide concentration in the reaction system is preferably increased up to 5 to 80 wt%, more preferably increased up to 5 to 60 wt%, and still more preferably increased up to 5 to 40 wt%, based on 100 wt% of the fluid containing cumene hydroperoxide.

In the step a4, a fluid containing cumene may be allowed to flow into the device A coincidently with the discharge of the fluid a'. The amount of the fluid containing cumene, which is allowed to flow into the device A, is not particularly limited, but the amount is preferably adjusted in such a manner that the liquid volume becomes constant.

After the start of the step a4, it is preferable to cool the reaction system by a heat exchange system X in such a manner that the temperature of the reaction system is maintained at a prescribed temperature. The temperature of the reaction system is not particularly limited, but it is usually 50 to 200°C, preferably 60 to 180°C, and more preferably 70 to 150°C. The pressure thereof is usually between atmospheric pressure and 5 MPa-G, preferably 0.01 to 2 MPa-G, and more preferably 0.02 to 1 MPa-G.

### EXAMPLES

Hereinafter, the present invention will be specifically described with examples, however, is in no way limited to the examples shown below.

### Example 1

A simulation using the device described in Invention 1 was carried out. As simulation software, DYNSIM Dynamic Simulation (AVEVA Group Limited) was used, and the amount of cumene hydroperoxide produced was determined by calculation referring to Journal of Chemical Engineering of Japan, 1970, 72.

A simulation model including one heat exchange system comprising a medium capable of both heating and cooling an oxidation reaction system, a medium cooling unit capable of cooling the medium, and a medium heating unit capable of heating the medium, and one oxidation reactor was prepared in the simulation software. In the reactor, 100 m³ of a 15% cumene hydroperoxide/cumene solution of 0.6 MPa-G was introduced. Using the medium heating unit side of the heat exchange system, the medium was heated, and using the heated medium, the oxidation reaction system was heated, thereby setting an internal temperature of the oxidation reactor to 90°C. Thereafter, not only was air having been heated to 90°C introduced at 500 Nm³/h, but also raw material cumene having been heated to 90°C was introduced at 4.8 ton/h, and coincidently, not only was unreacted gas discharged from the reactor, but also a reaction liquid was discharged in such a manner that the liquid volume in the reactor became 100 m³. The time at which introduction of air was started was taken as 0 hour, and an exit gas O₂ concentration of the reactor was confirmed every 6 hours, and when the O₂ concentration became 5% or less, the amount of air was increased by 500 Nm³/h, and the amount of the raw material cumene having been heated to 90°C was increased by 4.8 ton/h. Coincidently with a rise in internal temperature due to the heat of reaction, the medium heating unit used in the heat exchange system was switched to the medium cooling unit to cool the medium. Using the cooled medium, the oxidation reactor was cooled in such a manner that the internal temperature was adjusted to about 108°C. Thereafter, while the liquid volume in the reactor was maintained at 100 m³, the simulation was continued until the amount of air reached 2500 Nm³/h, the amount of the raw material cumene having been heated to 90°C reached 24 ton/h, and the temperature in the reactor became 108°C, and as a result, a steady state was achieved when the cumene hydroperoxide concentration in the reactor was about 13 wt%, and the exit gas O₂ concentration of the reactor was 2%. At this time, the time required for making the state steady from the start of introduction of air was 30 hours.

### Example 2

A simulation was carried out in the same manner as in Example 1, except that the time for confirming the exit gas O₂ concentration of the reactor was changed to every 3 hours. As a result, the time required for making the state steady was 15 hours.

### Example 3

A simulation was carried out in the same manner as in Example 1, except that the amounts of air introduced, the air having been heated to 90°C, and the raw material cumene introduced, the raw material cumene having been heated to 90°C, were changed to 1000 Nm³/h and 9.6 ton/h, respectively, and the increased amounts finally introduced were changed to 500 Nm³/h and 4.8 ton/h. As a result, the time required for making the state steady was 16 hours.

### Example 4

A simulation was carried out in the same manner as in Example 3, except that the temperatures of the heated air and the heated raw material cumene were each changed to 100°C. As a result, the time required for making the state steady was 16 hours.

### Example 5

A simulation model including one heat exchange system having a medium capable of both heating and cooling an oxidation reaction system, a medium cooling unit capable of cooling the medium, and a medium heating unit capable of heating the medium, and one oxidation reactor was prepared in the simulation software. In the reactor, 100 m³ of a 10% cumene hydroperoxide/cumene solution of 0.6 MPa-G was introduced. Using the medium heating unit side of the heat exchange system, the medium was heated, and using the heated medium, the oxidation reaction system was heated, thereby setting an internal temperature of the oxidation reactor to 90°C. Thereafter, not only was air having been heated to 90°C introduced at 370 Nm³/h, but also raw material cumene having been heated to 90°C was introduced at 4.8 ton/h, and coincidently, not only was unreacted gas discharged from the reactor, but also a reaction liquid was discharged in such a manner that the liquid volume in the reactor became 100 m³. The time at which introduction of air was started was taken as 0 hour, and an exit gas O₂ concentration of the reactor was confirmed every 3 hours, and when the O₂ concentration became 5% or less, the amount of air was increased by 370 Nm³/h, and the amount of the raw material cumene having been heated to 90°C was increased by 4.8 ton/h. Coincidently with a rise in internal temperature due to the heat of reaction, the medium heating unit used in the heat exchange system was switched to the medium cooling unit to cool the medium. Using the cooled medium, the oxidation reactor was cooled in such a manner that the internal temperature was adjusted to about 108 to 113°C. Thereafter, while the liquid volume in the reactor was maintained at 100 m³, the simulation was continued until the amount of air reached 2950 Nm³/h, the amount of the raw material cumene having been heated to 90°C reached 38.4 ton/h, and the temperature in the reactor became 113°C, and as a result, a steady state was achieved when the cumene hydroperoxide concentration in the reactor was about 10 wt%, and the exit gas O₂ concentration of the reactor was 2%. At this time, the time required for making the state steady from the start of introduction of air was 24 hours.

### Comparative Example 1

A simulation was carried out in the same manner as in Example 1, except that an oxidation reactor using a heat exchange system that did not heat a medium was assumed, and the reactor internal temperature before introduction of air was set to 25°C. As a result, the O₂ concentration did not become 5% or less within 30 hours, and the amount of air was unable to be increased. The reactor exit cumene hydroperoxide concentration after 30 hours was 2.5 wt%.

From the above, it can be seen that by using oxidation reaction equipment having a heat exchange system comprising a medium capable of both heating and cooling an oxidation reaction system, a medium cooling unit capable of cooling the medium, and a medium heating unit capable of heating the medium, the cumene hydroperoxide concentration in the reactor is allowed to easily reach a target concentration.

### INDUSTRIAL APPLICABILITY

The present invention is useful for producing cumene hydroperoxide. The present invention is also useful for producing propylene oxide.

### REFERENCE SIGNS LIST

1: cumene
2: oxygen-containing gas
3: fluid containing cumene hydroperoxide
4: alkaline reagent
5: discharged gas containing unreacted oxygen-containing gas
A: oxidation reactor
Xm: medium
Xh: medium heating unit (heat exchanger)
Xc: medium cooling unit (heat exchanger)
P: pump
V: valve

## Claims

1. Production equipment for producing cumene hydroperoxide by oxidizing cumene, comprising:
A: a device that oxidizes cumene to obtain cumene hydroperoxide; and
X: a heat exchange system,
wherein the heat exchange system X comprises a medium capable of both heating and cooling a reaction system for the oxidation, a medium cooling unit capable of cooling the medium, and a medium heating unit capable of heating the medium.

2. The production equipment according to claim 1, wherein the medium is a fluid containing water.

3. The production equipment according to claim 1 or 2, comprising
B: a device that allows cumene hydroperoxide to react with propylene to obtain propylene oxide and cumyl alcohol; and
E: a device that converts cumyl alcohol into cumene.

4. The production equipment according to any of claims 1 to 3, wherein the cooling of the medium is carried out by a fluid containing at least one selected from water, a process fluid of the device B, and a process fluid of the device E.

5. The production equipment according to any of claims 1 to 4, comprising at least one selected from
C: a device that separates propylene;
D: a device that separates propylene oxide; and
F: a device that purifies cumene.

6. The production equipment according to claim 5, wherein the cooling of the medium is carried out by a fluid containing at least one selected from water, a process fluid of the device C, a process fluid of the device D, and a process fluid of the device F.

7. The production equipment according to claim 6, wherein the cooling of the medium is carried out by a fluid containing at least one selected from water, a process fluid of the device C, and a process fluid of the device D.

8. The production equipment according to claim 7, wherein the device C comprises a device C' that separates propane, and the cooling of the medium is carried out by a fluid containing a process fluid of the device C'.

9. The production equipment according to claim 7 or 8, wherein the device D comprises a device D' that separates a heavy component, and the cooling of the medium is carried out by a fluid containing a process fluid of the device D'.

10. The production equipment according to any of claims 7 to 9, wherein the device D comprises a device D" that separates a light component, and the cooling of the medium is carried out by a fluid containing a process fluid of the device D".

11. The production equipment according to any of claims 1 to 10, wherein the heating of the medium is carried out by a fluid containing at least one selected from water (liquid) and water vapor.

12. The production equipment according to any of claims 1 to 11, comprising another cooling means than the heat exchange system X as a means for cooling the reaction system for the oxidation.

13. The production equipment according to claim 12, wherein the cooling means is an air fin cooler.

14. A method for producing cumene hydroperoxide, comprising the following steps:
a step of oxidizing cumene to obtain cumene hydroperoxide;
a step of heating a reaction system for the oxidation with a medium x;
a step of heating, in a heat exchange system X, the medium x having been cooled;
a step of cooling the reaction system for the oxidation with the medium x; and
a step of cooling, in the heat exchange system X, the medium x having been heated.
